**Europäisches Patentamt**

**European Patent Office**

**Office européen des brevets**

(19)

(11) Veröffentlichungsnummer: **0 059 775**
**B1**

(12) ## EUROPÄISCHE PATENTSCHRIFT

(45) Veröffentlichungstag der Patentschrift:
19.09.84

(21) Anmeldenummer: 81104171.4

(22) Anmeldetag: 01.06.81

(51) Int. Cl.³: **A 61 K 31/205**, A 61 K 31/70 //
(A61K31/70, 31/66, 31/195,
31/19)

(54) Infusionslösung zum Schutz der Leber und zur Verbesserung von deren Funktion und Verfahren zu deren Herstellung.

(30) Priorität: 09.06.80 YU 1533/80

(43) Veröffentlichungstag der Anmeldung:
15.09.82 Patentblatt 82/37

(45) Bekanntmachung des Hinweises auf die Patenterteilung:
19.09.84 Patentblatt 84/38

(84) Benannte Vertragsstaaten:
AT BE CH DE FR GB IT LI LU NL SE

(56) Entgegenhaltungen:
FR - A - 2 110 465
FR - A - 2 457 689

UNLISTED DRUGS, Band 25, Nr. 2, Februar 1973,
Chatham, N.J., USA
UNLISTED DRUGS, Band 18, Nr. 3, März 1966, Chatham,
N.J., USA

(73) Patentinhaber: **Leopold & Co. Chem. pharm. Fabrik
Gesellschaft m.b.H., Hafnerstrasse 36, A-8055 Graz (AT)**

(84) Benannte Vertragsstaaten: **BE CH FR GB IT LI LU NL SE
AT**

(73) Patentinhaber: **Lentia Gesellschaft mit beschränkter
Haftung, Arabellastrasse 4 Postfach 81 05 08,
D-8000 München 81 (DE)**

(84) Benannte Vertragsstaaten· **DE**

(72) Erfinder: **Jeretin, Stojan, Dr., Ferberjeva 37,
YU-6100 Ljubljana (YU)**

(74) Vertreter: **Allgeuer, Dorothea, Chemie Linz AG
Patentabteilung St. Peter-Strasse 25, A-4020 Linz (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft eine Infusionslösung zum Schutze der Leber bzw. zur Verbesserung von deren Funktion, die vor allem zur Behandlung von Leberinsuffizienz, bedingt durch Lebererkrankungen oder auch als Folge von Operationen, Trauma und Schock, geeignet ist.

Die Leber nimmt im Eiweiß- und Aminosäurestoffwechsel sowie bei der Entgiftung eine zentrale Stellung ein. Sie baut Eiweiß und Aminosäuren auf und ab, führt sie als Glykogen oder Fett in Depots über oder wandelt sie in andere Metaboliten um. Sie baut unter anderem die wichtigen Eiweißkörper Albumin und Globulin und auch die Gerinnungsfaktoren auf.

Bei Lebererkrankungen ist die Stoffwechselleistung eingeschränkt, die Desaminierung der Aminosäuren und die Harnstoffsynthese zum Teil gestört sowie die Synthese von Albumin, Globulin und der Gerinnungsfaktoren vermindert. Die Folge sind charakteristische Veränderungen des Plasmaaminosäurespiegels, ein Anstieg von Stoffwechselschlacken wie des Ammoniak und der Serumphenole, eine Verlängerung der Blutgerinnungszeit und ein Abfall wichtiger Plasma-Eiweißkörper.

Bei dieser verminderten Stoffwechselleistung werden üblicherweise Leberschutzlösungen verabreicht, die durch ihren Gehalt an Metaboliten des Harnstoff- und Zitratcyklus wie Arginin, Ornithin und Äpfelsäure diese beiden Cyklen stimulieren und helfen sollen, den Spiegel an Ammoniak und Phenolen im Serum zu senken. (Siehe z. B. R. Nordmann, Deutsche Med. Wochenschrift 94 (1969), 230—231.)

Es ist ferner bekannt, daß der Naturstoff Carnitin, nämlich $\beta$-Hydroxytrimethylammoniumbutyrobetain, eine Wirkung auf den Fettstoffwechsel in der Weise besitzt, daß damit die Lipidkonzentration im Blut gesenkt wird. Diese Wirkung beruht darauf, daß Carnitin den Transport von Fettsäuren in die Mitochondrien begünstigt. Carnitin wird daher schon zur Bekämpfung von Fettleibigkeit eingesetzt, andererseits dient es zur Verhütung von Degenerationserscheinungen, wie Arteriosklerose, die eine Folge einer erhöhten Konzentration von Lipiden im Blut ist (US-A 3 810 994). Für diesen Zweck wurde es auch schon zusammen mit fettspaltenden Agentien eingesetzt (DE-A 2 323 906). Weiters wurde die Verabreichung von Carnitin auch schon zur Behandlung von Herzarrhythmien und zur Verbesserung der Kontraktion des Herzmuskels (US-A 3 830 931 und US-A 3 968 241) sowie, und zwar vorzugsweise in Form eines Aerosols, zur Behandlung verschiedener Lungenerkrankungen vorgeschlagen (DE-A 2 360 332).

Es konnte nun überraschenderweise gefunden werden, daß Carnitin auch einen günstigen Einfluß auf den Leberstoffwechsel, vor allem jenen einer insuffizienten Leber besitzt. So ist es durch Verabreichung von Carnitin nicht nur möglich, eine durch Alkohol oder Glucose induzierte Leberverfettung zu verhindern, sondern es entfaltet auch eine Leberschutzwirkung, die sich sowohl durch eine überraschende Abnahme der Bilirubinwerte im Blut, als auch durch eine mit dem Quicktest meßbare Normalisierung der Blutgerinnungszeit leberinsuffizienter Patienten anzeigt, sofern Carnitin in ausreichend hohen Dosen verabreicht wird. Infusionslösungen, die Mengen an L-Carnitin innerhalb gewisser Grenzen neben den üblicherweise an leberinsuffiziente Patienten zu verabreichenden Metaboliten des Citrat- und des Harnstoffcyclus enthalten, sind daher besonders für die Behandlung und Besserung von Patienten mit Leberinsuffizienz geeignet. Nicht gemeint sind hier allerdings schwerste Leberfunktionsstörungen einschließlich des Coma hepaticum, für welchen Bereich andere Kriterien für die Behandlung gelten.

Gegenstand der vorliegenden Erfindung ist demnach eine für den genannten Zweck bestimmte Infusionslösung, die L-Arginin, Äpfelsäure, L-Asparaginsäure, Malat-ionen und Kohlenhydrate enthält, die dadurch gekennzeichnet ist, daß sie 2—15 g/l L-Carnitin enthält.

Besonders bevorzugt sind hierbei Lösungen, die außer der erfindungsgemäßen Menge an L-Carnitin die oben genannten weiteren Bestandteile im folgenden Mengenverhältnis enthalten

| | |
|---|---|
| L-Arginin | 3— 20 g/l |
| Äpfelsäure, zum Teil als Malat | 1— 10 g/l |
| L-Asparaginsäure | 1— 6 g/l |
| Glucose | 50—100 g/l |

Die Beigabe von Glucose anstelle anderer Zucker oder der üblichen Zuckerersatzstoffe wie Xylit oder Sorbit, ist deshalb bevorzugt, weil Glucose das physiologische Substrat für die primäre Energiebereitstellung ist und ubiquitär, also überwiegend außerhalb der Leber, verwertet wird. Die in der Infusionstherapie häufig verwendeten Glucoseersatzstoffe Laevulose, Sorbit und Xylit hingegen, werden überwiegend in der Leber metabolisiert, weshalb ihre Verwertung bei Leberinsuffizienz gestört ist. Ihre Verwendung ist daher für den erfindungsgemäßen Zweck weniger angezeigt.

In den erfindungsgemäßen Lösungen kann ein Teil des Arginins auch durch eine äquivalente Menge an Ornithin ersetzt sein, das ebenfalls ein Metabolit im Harnstoffcyclus ist.

Außer den erfindungsgemäßen Bestandteilen können die erfindungsgemäßen Infusionslösungen auch die üblichen Zusätze wie Vitamine, weitere Aminosäuren, und ähnliches enthalten. Besonders hervorzuheben sind hierbei Elektrolyte wie Na, K, Ca, Mg und Zn-Salze sowie Phosphate. Im letzteren Falle sind vor allem organische Phosphate bevorzugt, die, wie die Glucosephosphate oder Fructosephosphate im Organismus als Bestandteil des Kohlehydratstoffwechsels vorkommen oder diesen, wie die Glycerophosphate, nahe verwandt sind. Der Zusatz von Phosphat in dieser Form hat den Vorteil, daß auch bei Verwendung von Ca- und/oder Mg-Verbindungen keine schwerlöslichen Niederschläge auftreten.

Die Herstellung der erfindungsgemäßen Infusionslösungen erfolgt auf übliche Weise. Zweckmäßig werden das L-Carnitin sowie die Aminosäuren Arginin, Asparaginsäure und gegebenenfalls Ornithin gleichzeitig oder aufeinanderfolgend unter Rühren in warmem Wasser gelöst. Anschließend werden die Äpfelsäure, gegebenenfalls Alkalilauge zur teilweisen Neutralisation der Äpfelsäure sowie ev. vorhandene andere Bestandteile zugegeben, worauf in der Lösung nötigenfalls ein pH-Wert innerhalb des physiologischen Bereiches eingestellt wird. Dies geschieht am besten durch entsprechende Wahl des Verhältnisses zwischen Äpfelsäure und Malat-ionen. Schließlich wird mit Wasser ad infundi auf das gewünschte Volumen aufgefüllt.

Die Maillard'sche Reaktion zwischen Glucose und den Aminosäuren kann vermieden werden, wenn für die Sterilisierung eine entsprechende Vorgangsweise gewählt wird. Vorzugsweise werden diese Lösungen steril filtriert und aseptisch abgefüllt.

Die erfindungsgemäßen Lösungen werden vor allem Patienten verabreicht, deren Leberfunktion nach Operationen, Trauma oder Schock geschädigt ist. Durch Relaxierung, Anaesthesie und Sedativa, ferner durch Minderperfusion und Hypoxie der Leber, sowie durch Resorption von Hämatomen und Eiweißfragmenten, manchmal auch durch falsch konzipierte parenterale Ernährung kommt es zur Überlastung von Entgiftungs- und Syntheseleistungen der Leberzellen, die durch die Verabreichung der erfindungsgemäßen Infusionslösungen entlastet und damit geschützt werden, was durch die Normalisierung der Bilirubin- und Quickwerte festgestellt werden kann. Die erfindungsgemäßen Infusionslösungen können aber auch bei solchen Fällen von Hepatitis und Leberzirrhose angewendet werden, bei denen noch eine ausreichende Restfunktion der Leber gegeben ist.

Wesentlich hierbei ist aber, daß die Konzentration des Carnitins in der Lösung 2 g/l Infusionslösung nicht unterschreitet.

Dies ist begründet in der Kinetik des Carnitins, das für den 70 kg schweren Patienten einen Verteilungsraum von ca. 40 l und eine Halbwertszeit von 4—5 Stunden besitzt, wobei im inrazellulären Kompartiment jedoch eine bis zu 60fach höhere Konzentration wie im Extrazellulärraum angetroffen wird. Unter Berücksichtigung der Tatsache, daß im pathologischen Zustand die Syntheserate des Carnitins in der Leber stark vermindert ist, ist eine Zufuhr der erfindungsgemäßen Lösung in einer Menge, die einer täglichen Dosis an Carnitin von 2—15 g entspricht, zu empfehlen, wobei für Patienten mit Leberzirrhose vor allem die Dosen im oberen Teil des angegebenen Bereiches in Frage kommen.

Die folgenden Beispiele sollen die vorliegende Erfindung näher erläutern, ohne sie zu beschränken.

### Beispiel 1

Eine Lösung folgender Zusammensetzung wird bereitet:

| | |
|---|---|
| NaOH | 1,408 g/l |
| Na-Acetat · 3 HOH | 2,286 g/l |
| KCl | 2,400 g/l |
| MgCl$_2$ · 6 HOH | 1,017 g/l |
| L-Ornithin · HCl | 8,228 g/l |
| L-Asparaginsäure | 3,565 g/l |
| L-Carnitin | 6,000 g/l |
| Glucose | 100,000 g/l |
| L-Arginin | 3,000 g/l |
| L-Äpfelsäure | 2,300 g/l |
| aqua ad infundi | ad 1000 ml |

Zunächst werden die Aminosäuren und das L-Carnitin in Wasser von 60°C unter Rühren gelöst. Nach Abkühlen werden die Äpfelsäure, das Natriumhydroxyd und die Elektrolyte und schließlich die Glucose zugesetzt und auf ein Volumen von 1 Liter aufgefüllt. Die Sterilisierung erfolgt durch Sterilfiltration.

### Beispiel 2

Eine Lösung folgender Zusammensetzung wird, wie in Beispiel 1 beschrieben, bereitet:

| | |
|---|---|
| L-Arginin · HCl | 1,264 g/l |
| L-Arginin · Base | 4,955 g/l |
| L-Asparaginsäure | 6,000 g/l |
| L-Carnitin | 10,000 g/l |
| L-Äpfelsäure | 2,500 g/l |
| Glucose | 50,000 g/l |
| NaCl | 1,100 g/l |
| Na-Acetat · 3 HOH | 2,340 g/l |
| Na$_2$-Glycerophosphat · 5 HOH | 1,837 g/l |
| KCl | 0,716 g/l |
| CaCl$_2$ · 2 HOH | 0,176 g/l |
| MgCl$_2$ · 6 HOH | 0,325 g/l |
| ZnCl$_2$ | 0,007 g/l |
| aqua ad infundi | ad 1000 ml |

### Beispiel 3

Wie in Beispiel 1 beschrieben wird eine Lösung folgender Zusammensetzung bereitet:

| | |
|---|---|
| L-Arginin · Base | 3,000 g/l |
| L-Asparaginsäure | 6,000 g/l |
| L-Carnitin | 15,000 g/l |
| L-Äpfelsäure | 1,500 g/l |
| Glucose | 50,000 g/l |
| NaCl | 1,450 g/l |
| Na-Acetat · 3 HOH | 2,340 g/l |
| KCl | 0,716 g/l |
| CaCl$_2$ · 2 HOH | 0,176 g/l |
| MgCl$_2$ · 6 HOH | 0,325 g/l |
| ZnCl$_2$ | 0,007 g/l |
| aqua ad infundi | ad 1000 ml |

## Patentansprüche für die Vertragsstaaten BE, CH, DE, FR, GB, IT, LI, LU, NL, SE

1. Infusionslösung zum Schutz der Leber und zur Verbesserung von deren Funktion enthaltend L-Arginin, Äpfelsäure, L-Asparaginsäure, Malat-ionen und Kohlenhydrate, dadurch gekennzeichnet, daß sie zusätzlich 2—15 g/l L-Carnitin ($\beta$-Hydroxytrimethylammonium-butyrobetain) enthält.

2. Infusionslösung nach Anspruch 1, dadurch gekennzeichnet, daß sie

| | | |
|---|---|---|
| 3— | 20 g/l | L-Arginin |
| 1— | 10 g/l | Äpfelsäure, zum Teil als Malat |
| 1— | 6 g/l | L-Asparaginsäure |
| 2— | 15 g/l | L-Carnitin und |
| 50—100 g/l | | Glucose |

enthält.

3. Infusionslösung nach den Ansprüchen 1 und 2, dadurch gekennzeichnet, daß ein Teil des Arginins durch die äquivalente Menge Ornithin ersetzt ist.

4. Infusionslösung nach den Ansprüchen 1 bis 3, dadurch gekennzeichnet, daß sie zusätzlich Elektrolyte enthält.

5. Infusionslösung nach den Ansprüchen 1 bis 4, dadurch gekennzeichnet, daß sie zusätzlich im menschlichen Organismus vorkommende organische Phosphate enthält, die entweder Intermediärprodukte des menschlichen Kohlehydratstoffwechsels oder diesem nahe verwandt sind.

6. Verfahren zur Herstellung von Infusionslösungen gemäß den Ansprüchen 1 bis 5, dadurch gekennzeichnet, daß das Arginin, die Asparaginsäure und gegebenenfalls das Ornithin sowie L-Carnitin in Form seines inneren Salzes oder eines pharmazeutisch verträglichen Säureadditionssalzes gemeinsam oder aufeinanderfolgend in warmem Wasser gelöst werden, worauf die Äpfelsäure und die Kohlenhydrate vorzugsweise Glucose sowie gegebenenfalls Elektrolyte, im menschlichen Organismus vorkommende organische Phosphate, die entweder Intermediärprodukte des menschlichen Kohlehydratstoffwechsels oder diesem nahe verwandt sind, sowie gewünschtenfalls andere übliche Zusätze hinzugefügt werden und die Lösung nach gegebenenfalls erforderlicher Einstellung eines pH-Wertes innerhalb des physiologischen Bereiches mit Wasser auf das gewünschte Volumen aufgefüllt wird, wobei die Menge an L-Carnitin 2 – 15 g/l beträgt.

## Patentanspruch für den Vertragsstaat AT

Verfahren zur Herstellung einer Infusionslösung zum Schutz der Leber und zur Verbesserung von deren Funktion enthaltend L-Arginin, Äpfelsäure, L-Asparaginsäure, Malat-ionen und Kohlenhydrate, dadurch gekennzeichnet, daß das L-Arginin, die L-Asparaginsäure und gegebenenfalls L-Ornithin sowie L-Carnitin ($\beta$-Hydro-xytrimethylammonium-butyrobetain) in Form seines inneren Salzes oder eines pharmazeutisch verträglichen Säureadditionssalzes gemeinsam oder aufeinanderfolgend in warmem Wasser gelöst werden, worauf die Äpfelsäure und die Kohlenhydrate, vorzugsweise Glucose sowie gegebenenfalls Elektrolyte, im menschlichen Organismus vorkommende organische Phosphate, die entweder Intermediärprodukte des menschlichen Kohlehydratstoffwechsels oder diesem nahe verwandt sind, sowie gewünschtenfalls andere übliche Zusätze hinzugefügt werden und die Lösung nach gegebenenfalls erforderlicher Einstellung eines pH-Wertes innerhalb des physiologischen Bereiches mit Wasser auf das gewünschte Volumen aufgefüllt wird, wobei die Menge an L-Carnitin 2—15 g/l beträgt.

## Claims for the Contracting States: BE, CH, DE, FR, GB, IT, LI, LU, NL

1. Infusion solution, for protecting the liver and for improving its function, containing L-arginine, malic acid, L-asparaginic acid, malate ions and carbohydrates, characterized in that it additionally contains 2—15 g/litre of L-carnitine ($\beta$-hydroxytrimethylammonium-butyrobetaine).

2. Infusion solution according to claim 1, characterized in that it contains

| | | |
|---|---|---|
| 3— | 20 g/litre | L-arginine, |
| 1— | 10 g/litre | Malic acid, partly as malate |
| 1— | 6 g/litre | L-asparaginic acid |
| 2— | 15 g/litre | L-carnitine and |
| 50—100 g/litre | | Glucose. |

3. Infusion solution according to claims 1 and 2, characterized in that some of the arginine is replaced by an equivalent amount of ornithine.

4. Infusion solution according to claims 1 to 3, characterized in that it additionally contains electrolytes.

5. Infusion solution according to claims 1 to 4, characterized in that it additionally contains organic phosphates which occur in the human organism and are either intermediate products of human carbohydrate metabolism or are closely related to these.

6. Process for the preparation of infusion solutions according to claims 1 to 5, characterized in that the arginine, the asparaginic acid and where relevant the ornithine and L-carnitine in the form of its inner salt or a pharmaceutically acceptable acid addition salt are dissolved, together or successively, in warm water, after which the malic acid and the carbohydrates, preferably glucose, and, if appropriate, electrolytes, organic phosphates which occur in the human organism and are either intermediate products of human carbohydrate metabolism or are closely related to these, and, if desired, other conventional additives are added and, after any necessary adjustment of the pH value to within the physiological range, the solution is made up to the desired volume with water, the amount of L-carnitine being 2—15 g/litre.

## Claim for the contracting State: AT

Process for the preparation of an infusion solution, for protecting the liver and for improving its function, containing L-arginine, malic acid, L-asparaginic acid, malate ions and carbohydrates, characterized in that the L-arginine, the L-asparaginic acid and, where relevant, L-ornithine and L-carnitine (β-hydroxytrimethyl-ammonium-butyrobetaine) in the form of its inner salt or a pharmaceutically acceptable acid addition salt are dissolved, together or successively, in warm water, after which the malic acid and the carbohydrates, preferably glucose, and, if appropriate, electrolytes, organic phosphates which occur in the human organism and are either intermediate products of human carbohydrate metabolism or are closely related to these, and, if desired, other conventional additives are added and, after any necessary adjustment of the pH value to within the physiological range, the solution is made up to the desired volume with water, the amount of L-carnitine being 2—15 g/litre.

## Revendications pour les Etats contractants: BE, CH, DE, FR, GB, IT, LI, LU, NL

1. Solution pour infusion pour la protection du foie et pour l'amélioration de sa fonction, contenant de la L-arginine, de l'acide malique, de l'acide L-aspartique, des ions malate et des hydrates de carbone, caractérisée en ce qu'elle renferme en outre de 2 à 15 g/l de L-carnitine (β-hydroxytriméthylammonium-butyrobétaïne).

2. Solution pour injection suivant la revendication 1, caractérisée en ce qu'elle renferme:

| | |
|---|---|
| 3— 20 g/l | de L-arginine, |
| 1— 10 g/l | d'acide malique, en partie sous forme de malate, |
| 1— 6 g/l | d'acide L-aspartique, |
| 2— 15 g/l | de L-carnitine, et |
| 50—100 g/l | de glucose. |

3. Solution pour infusion suivant les revendications 1 et 2, caractérisée en ce qu'une partie de l'arginine est remplacée par la quantité équivalente d'ornithine.

4. Solution pour infusion suivant les revendications 1 à 3, caractérisée en ce qu'elle renferme en outre des électrolytes.

5. Solution pour infusion suivant les revendications 1 à 4, caractérisée en ce qu'elle renferme en outre des phosphates organiques présents dans l'organisme humain, qui sont constitués soit par des produits intermédiaires du métabolisme humain des hydrates de carbone, soit par des produits étroitement apparentés à ceux-ci.

6. Procédé pour la préparation de solutions pour infusion suivant les revendications 1 à 5, caractérisé en ce qu'on fait dissoudre l'arginine, l'acide aspartique et le cas échéant l'ornithine, ainsi que la L-carnitine sous forme de son sel interne ou d'un sel d'addition avec un acide pharmaceutiquement acceptable dans l'eau chaude, conjointement ou de façon successive, après quoi on ajoute l'acide malique et les hydrates de carbone, de préférence du glucose, ainsi que le cas échéant des électrolytes, des phosphates organiques présents dans l'organisme humain, qui sont soit des produits intermédiaires du métabolisme humain des hydrates de carbone, soit des produits étroitement apparentés à ceux-ci, ainsi que si désiré d'autres additifs usuels, et on complète la solution au volume désiré avec de l'eau, le cas échéant après réglage nécessaire d'un pH compris dans la gamme physiologique, la quantité de L-carnitine représentant de 2 à 15 g/l.

## Revendication pour l'Etats contractant: AT

Procédé pour la préparation d'une solution pour infusion pour la protection du foie et pour l'amélioration de sa fonction, contenant de la L-arginine, de l'acide malique, de l'acide L-aspartique, des ions malate et des hydrates de carbone, caractérisé en ce qu'on fait dissoudre la L-arginine, l'acide L-aspartique et le cas échéant de la L-ornithine, ainsi que de la L-carnitine (β-hydroxytriméthylammonium-butyrobétaïne) sous forme de son sel interne ou d'un sel d'addition avec un acide pharmaceutiquement acceptable dans de l'eau chaude, conjointement ou de façon successive, après quoi on ajoute l'acide malique et les hydrates de carbone, de préférence du glucose, ainsi que le cas échéant des électrolytes, des phosphates organiques présents dans l'organisme humain, qui sont soit des produits intermédiaires du métabolisme humain des hydrates de carbone, soit des produits étroitement apparentés à ceux-ci, ainsi que si désiré d'autres additifs usuels, et on complète la solution au volume désiré avec de l'eau, éventuellement si nécessaire après réglage d'un pH compris dans la gamme physiologique, la quantité de L-carnitine représentant de 2 à 15 g/l.